# EUROPÄISCHE PATENTSCHRIFT

(11) **EP 2 068 815 B1**
(45) Veröffentlichungstag und Bekanntmachung des Hinweises auf die Patenterteilung: **22.12.2010**
(21) Anmeldenummer: 07820823.8
(22) Anmeldetag: 02.10.2007
(51) Int. Cl.: A61K 8/25, A61K 8/24, A61K 8/27, A61Q 11/00

(54) **DENTALE, INSBESONDERE REMINERALISIERENDE UND GEGEN SCHMERZEMPFINDLICHE ZÄHNE WIRKSAME ZUSAMMENSETZUNG SOWIE DENTALE PARTIKEL, INSBESONDERE FÜR DIE ZUSAMMENSETZUNG**
DENTAL, PARTICULARLY REMINERALIZING COMPOSITION, EFFECTIVE FOR PAIN SENSITIVE TEETH, AND DENTAL PARTICLES, PARTICULARLY FOR SAID COMPOSITION
COMPOSITION DENTAIRE, EN PARTICULIER REMINÉRALISANTE ET EFFICACE CONTRE LES DENTS SENSIBLES, AINSI QUE PARTICULES DENTAIRES, EN PARTICULIER POUR LA COMPOSITION

(30) Priorität: 04.10.2006 DE 102006046952
(43) Veröffentlichungstag der Anmeldung: 17.06.2009
(73) Patentinhaber: Ley, Fritz, 53507 Dernau (DE)
(72) Erfinder: CURRO, Frederick, Emerson, New Jersey 07630 (US); ALBERT, Heidi, 72076 Tübingen (DE); LEY, Fritz, 53507 Dernau (DE)
(74) Vertreter: Solf, Alexander
(86) Internationale Anmeldenummer: PCT/EP2007/060441
(87) Internationale Veröffentlichungsnummer: WO 2008/040723

(56) Entgegenhaltungen:
- WO-A-00/59460
- WO-A-02/30380
- WO-A-2005/002722
- US-A- 6 086 374

## Beschreibung

Die Erfindung betrifft sphärische Dentalpartikel, insbesondere für remineralisierende und gegen schmerzempfindliche Zähne wirksame Zusammensetzung, wie Zahnpasta, Zahnpulver, Mundwasser, Kaugummi, zahnärztliche therapeutische Beschichtungen oder dergleichen. Die Erfindung betrifft zudem dentale, die Dentalpartikel, aufweisende Zusammensetzungen.

Die Oberfläche von Zähnen befindet sich in einem konstanten Gleichgewicht zwischen dem Verlust und der Aufnahme von Mineralien. Dieser Prozess wird teilweise durch die chemische Zusammensetzung des Speichels und der extrazellulären Flüssigkeit im Gleichgewicht gehalten. Eine Störung der Integrität der Zahnoberflächen kann z. B. durch saure Nahrungsmittel und Getränke, durch bakterielle Belastung und durch Erosion wegen übermäßigen Zähneputzens und auch Zähneknirschens hervorgerufen werden.

Diese Prozesse werden von einer Demineralisierung der exponierten Zahnoberflächen begleitet, die zu klinischen Zuständen wie empfindlichen Zähnen und Karies führen.

Zahnschmelz besteht überwiegend aus anorganischer mineralischer Substanz (95 Gewichtsprozent = 86 Volumenprozent). Er besteht nicht aus reinem Hydroxylapatit ((Ca10P04)6(OH)2), sondern aus Misch- und Defektapatit. Beim Mischapatit können an die Stelle der Calcium-Ionen Magnesium, Strontium- oder andere zweifach positiv geladene Ionen treten, an die Stelle des PO4³⁻kann HPO4²⁻, H₂PO4⁻, CO₃²⁻ oder HCO₃⁻ treten. Beim Defektapatit bleibt die Stelle des Calcium-Ions im Kristallgitter unbesetzt.

Im Speichel sind Mineralien wie Calcium und Phosphate gelöst. Bei sinkendem pH in der Plaque und im Speichel werden die dort gelösten Phosphat-Ionen protoniert und so dem Lösungsgleichgewicht entzogen. Um das Gleichgewicht wiederherzustellen, müssen Apatitkristalle aus dem Schmelz gelöst werden. Daher kommt es zur Demineralisation des Schmelzes (Initialläsion). Steigt der pH, kommt es zur umgekehrten Reaktion. Da der Speichel dann bezogen auf Apatit übersättigt ist, es kommt zur Remineralisation.

Unmittelbar über dem noch gesunden Zahnschmelz, befindet sich in einer Initialläsion eine transluzente Zone. Sie enthält ca. 1% Poren. Gesunder Schmelz enthält dagegen nur etwa 0,1% Poren. Diese Poren oder Mikrokanäle besitzen einen Durchmesser von etwa 0.5 - 1.5 Mikrometern und eine Länge von ungefähr 100 Mikrometern, die schließlich ein Eindringen von Bakterien ermöglichen.

Durch eine Demineralisierung und durch Bakterien kann also im Schmelz und auch im Dentin Karies auftreten. Schmerzempfindlichkeit der Zähne ist ein klinischer Zustand, der durch eine übermäßige Reaktion der Pulpa auf Reize wie Kälte, Süβes usw. definiert ist. Die Ursache der übermäßigen Reaktion ist freiliegendes Dentin, das durch Erosion von Schmelz/Zementum, durch Rückgang des Zahnfleisches und/oder durch parodontal-chirurgische Eingriffe exponiert wird.

Der mineralische Anteil des Dentins liegt bei "lediglich" 70 Gewichtsprozent bzw. 45 Volumenprozent. Die Auflösung des Dentins beginnt bereits bei einem höheren pH als die Auflösung des Schmelzes und es ist außerdem wesentlich poröser. Daher breitet sich eine Karies nach dem Erreichen des Dentins wesentlich schneller als im Schmelz. Der Körper reagiert mit einer Sklerosierung des Dentins als Versuch eine Barriere zwischen Karies und Pulpa aufzubauen. In den Tubuli werden Mineralien abgelagert, die eine Diffusion von Säuren, toxischen Substanzen und proteolytischen Enzymen ebenso hemmen wie das Vordringen von Bakterien.

Es wurden viele Ansätze unternommen, die Dentinhypersensitivität, zu kontrollieren. Ein Ansatz im Stand der Technik besteht darin, die Empfindlichkeit des Zahnnervs in dem sensiblen Zahn durch Verändern der chemischen Umgebung zu verringern. Hierzu werden Agentien verwendet, die den Zahn weniger sensibel machen. Im Stand der Technik sind verschiedene so genannte Nerv desensibilisierende Agentien bekannt. Zu diesem Zweck wird überwiegend Kaliumnitrat verwendet, das in kommerziellen Zahnpasten zur Desensibilisierung von Zähnen eingesetzt wird (US-Patent 3,863,006, 4,009,327 und 4,751,072). Dort werden überwiegend Formulierungen bzw. Zusammensetzungen beschrieben, die Kaliumsalze, wie auch Kaliumbicarbonat und Kaliumchlorid enthalten.

Ein anderer Ansatz im Stand der Technik besteht darin, die Dentinhypersensitivität zu kontrollieren durch Verwendung von Agentien, die die Tubuli teilweise oder ganz verschließen. Diese Agentien werden als Tubuli-blockierende Agentien bezeichnet. Im US-Patent 4,990,327 wird eine Desensibilisierung von Zähnen mit Strontium- und Fluoridionen offenbart. Des Weiteren offenbart US-Patent 3,888,976 die Behandlung von sensitiven Zähnen durch Verwendung von Zink- und Strontiumionen. Das US-Patent 5,211,937 berichtet über die Verwendung von geladenen Polystyrol-Kügelchen als chemisch inertes Agens, das mechanisch die Oberfläche der Tubuli blockiert. US-Patente 4,634,589 und 4,710,372 befassen sich mit der Verwendung von Tonen wie Laponit oder Hectorit, um die Tubuli zu versiegeln. US-Patent 5,270,031 beschreibt die Verwendung von Polyacrylsäuren, die ein typisches Molekulargewicht von ungefähr 450.000 bis ungefähr 4.000.000 aufweisen und als Tubuli blockierendes Agens verwendet werden. Weiterhin wird im US-Patent 4,362,713 die Verwendung von wasserlöslichen oder wasserquellbaren Polyelektrolyten und deren Salze als Tubuli blockierende Agentien beschrieben.

Darüber hinaus ist im Stand der Technik die Verwendung von Substraten in Form von sphärischen Mikropartikeln bekannt, auf welchen eine aktive chemotherapeutische Substanz durch chemische, elektrostatische oder ionische Bindung adsorbiert ist, um die Rate der Wundheilung oder Knochenregeneration zu beschleunigen. Dadurch können aktive chemische Substanzen zur Behandlung kontrolliert freigesetzt werden. Diese so genannten Mikrosphären können hohl oder massiv sein; die chemische Substanz kann auf der Oberfläche adsorbiert oder darin verkapselt vorliegen.

Aus der US-PS 5,037,639 ist die Verwendung von amorphen, also nicht sphärischen Calciumverbindungen bekannt, die auf die Dentinstruktur der Zähne aufgetragen werden, wonach sich Apatit in situ bilden soll. Diese Apatitbildung soll zu einer Remineralisierung der Zähne und Verringerung der Dentinüberempfindlichkeit führen.

Die DE 695 24 747 T2 enthält eine umfangreiche Übersicht über den Stand der Technik bezüglich der Linderung von Dentinüberempfindlichkeit mit Submikron-Partikeln und beschreibt die Verwendung von kationisch geladenen kolloidalen Partikeln in wässriger Umgebung. Beispielsweise wird vorgeschlagen, handelsübliches mit Aluminiumoxid einer Partikelgröße von 2 nm beschichtetes, nicht sphärisches Siliziumdioxid zu verwenden, das mittlere Partikelgrößen von 20 nm aufweist. Dieses Produkt liegt in Form einer wässrigen kolloidalen Dispersion der Aluminium-Submikronen-Teilchen vor. Für dentale Zusammensetzungen soll das Produkt bzw. Mittel in Mengen von 0,1 - 10 Gew.-% eingesetzt werden.

In der Dissertation von Gerd Fischer über das Thema "Entwicklung, Charakterisierung und Anwendung neuer Hybridmaterialien", vorgelegt der Fakultät für Chemie und Pharmazie der Eberhard-Karls-Universität Tübingen, 2004, wird über die steuerbare Herstellung von monodispersen sphärischen Kieselgelpartikeln der Größenordnung zwischen etwa 0,2 und 1,8 µm nach dem Sol-Gel-Verfahren und deren Kombination mit anderen Materialien berichtet. Unter anderem wird berichtet über die Ummantelung und die Co-Kondensation mit Hydroxyl-Apatit, woraus monodisperse Partikel mit Korngrößen zwischen 1,5 und 3,5 µm resultieren.

In der Kombination beider Materialien sollen die Kieselgelpartikel Form und Größe und Hydroxyl-Apatit die Ähnlichkeit zum Zahnmaterial einbringen, wobei an den Aufbau von Zahnschmelzverlusten durch die wirkung des Hydroxyl-Apatits gedacht ist.

In der EP 0 216 278 B1 wird die Herstellung von unporösen bzw. möglichst keine Porosität aufweisenden monodispersen kugelförmigen bzw. sphärischen Kieselgelpartikeln nach dem Sol-Gel-Verfahren beschrieben, wobei insbesondere Partikel mit Partikelgrößen von 0,05 bis 10 µm hergestellt werden.

Die DE 103 30 204 A1 beschreibt ein Sol-Gel-Verfahren zur Herstellung von unporösen sphärischen monodispersen Kieselgelpartikeln, insbesondere einer Teilchengröße zwischen 0,2 und 1,2 µm, die über einen so genannten Spacer immobilisierende Moleküle auf ihrer Oberfläche tragen.

Darüber hinaus befasst sich die WO 02/30380 A1 mit der Desensibilisierung von Zähnen u.a. durch die Einleitung einer Regeneration des Knochenmaterials zur Schließung der Dentin-Tubuli. Vorgeschlagen wird u.a. die Verwendung von Hydroxyl-Apatit, Fluor-Apatit, Chlor-Apatit, Tri-Calciumphosphat und anderen auf Calciumphosphaten basierenden Verbindungen. Diese Verbindungen können u.a. auf einem Substrat aus Kieselgel vorgesehen sein, wobei die Teilchengröße des Substrats < 10 Mikrons sein soll. Die die desensibilisierende Verbindung tragenden porösen oder nicht porösen Teilchen bzw. Partikel sollen in einer Menge von 1 bis 70 Gew.-% in dentalen Zusammensetzungen wie zahnärztlichen bzw. desensibilisierenden Zusammensetzungen wie Zahnpasta, Mundwasser, Zahnpulver, Beschichtungen, Kaugummi oder dergleichen vorhanden sein. Die Zusammensetzungen können darüber hinaus weitere üblicherweise verwendete z. B. abrasive Bestandteile und auch weitere desensibilisierende oder anderweitig wirkende Mittel aufweisen.

Nachteilig an diesen im Stand der Technik bekannten Agentien zur Desensibilisierung der Zähne ist, dass sie lediglich temporär die Symptome lindern, ohne dauerhaft zu einem Verschluss der offenen Tubuli durch Einlagerung und Remineralisierung d.h. Kristallwachstum zu führen, da sie nur an der Oberfläche wirken. Nur ein dauerhafter Verschluss der Tubuli kann das Auftreten schmerzempfindlicher Zähne verhindern.

Diese im Stand der Technik verwendeten oberflächenwirksamen Substanzen vermögen dies nicht zu leisten. Diese Substanzen dringen nur unzureichend oder gar nicht in die Zahntubuli ein und vermögen nicht die Reizweiterleitung in den Nerv tatsächlich zu beeinflussen d.h. zu hemmen. Aufgrund der chemischen Struktur dieser Substanzen können sie nicht oder nur unzureichend in die bestehende Zahnstruktur eingebaut werden, um so eine ausreichende Remineralisierung der Zähne und einen Verschluss der Tubuli zu bewirken.

Dies gilt jedoch nicht ausschließlich für das Dentin, sondern auch für den Schmelz. Der Stand der Technik beschreibt eine Vielzahl von Patenten, die eine Remineralisierung von Schmelzläsionen und eine Prävention von Karies beschreiben. US Patent 3,175,951 beschreibt beispielsweise die Verwendung von Fluoriden bei der Bekämpfung von Karies.

In vitro und in vivo konnte gezeigt werden, dass Initialläsionen durch Anwendung von Fluorid zumindest teilweise remineralisiert und ggf. sogar komplett wiederhergestellt wurden. Fluorid kann das aus dem Gleichgewicht geratene Wechselspiel von De- und Remineralisation zugunsten der Remineralisation beeinflussen. Über Diffusionskanäle dringt Fluorid in den Schmelz ein. Dabei entsteht stabil in der Zahnhartsubstanz gebundenes Fluorid, da Fluorapatit oder fluoridierter Hydroxylapatit gebildet wird. In der äußerten Schmelzschicht sind weniger als 10% der OH⁻-Gruppen durch Fluorid ersetzt, in einer Tiefe von 50 µm sind es bereits nur noch 1%. Während im Speichel Hydroxylapatit bereits bei einem pH-Wert von etwa 5,5 demineralisiert wird, geschieht das für Fluorapatit erst bei einem pH-Wert von 4,6. Bei der umgekehrten Reaktion, also der Remineralisation, bedeutet dies, daß bei Anwesenheit von ausreichend Fluorid idealerweise bereits bei einem pH-Wert von 4,6 eine Remineralisation einsetzen kann, während das ohne Fluorid erst bei pH 5,5 der Fall ist.

Eine weitere Methode der Förderung der Remineralisation besteht in der Anwendung von löslichen Calciumsalzen. US Patent 4,080,440 beschreibt die Verwendung von löslichen Calciumsalzen mit löslichen Fluoriden zur Remineralisierung von Schmelz. US Patent 4,048,300 beschreibt die Verwendung von Calciumphosphaten wie Fluorapatit, Fluorhydroxyapatit und Hydroxyapatit sowie Monofluorphosphat und Carbonat und zweiwertigen Kationen wie Zink.

Die US Patente 6,733,818 und 6,846,500 kombinieren amorphes Calciumphosphat mit Casein-phosphopeptid sowie Bicarbonat als Kaugummis oder Süßigkeiten. US Patente 5,858,333 und 5,603,922 beschreiben die Verwendung von Produkten in 2-Kammer-Tuben, in deren einem Teil sich lösliche Calciumsalze und in den anderen sich Phosphate und Fluoride befinden, die sich bei simultanem Herauspressen mischen und dann in situ zu einer Remineralisierung führen. US Patente 5,534,244, 5,437,857 und 5,460,803 beschreiben die Verwendung von amorphen Strontium- und Calciumphosphaten mit oder ohne Fluorid zur Remineralisierung von Schmelz.

United States Patent 6,521,215 beschreibt vorteilhafte Zusammensetzungen und Methoden zum Bleichen und Remineralisieren von Zähnen gegen schmerzempfindliche Zähne und für die Behandlung von Karies mit einem löslichen Calciumphosphat bestehend aus einem oder mehreren der Gruppe Monocalciumphosphat, Tricalciumphosphat und Tetracalciumphosphat. Das US Patent 4,923,683 beschreibt die Anwendung von mikroverkapseltem Hydroxylapatit und / oder Fluorid.

Aufgabe der Erfindung ist es, Dentalpartikel und dentale Zusammensetzungen zu entwickeln, die zum einen ausreichend in das Zahnmaterial eindringen können um effizient zur Remineralisierung der Zahnstruktur des Schmelzes und des Dentins beizutragen und / oder durch Remineralisierung und Kristallwachstum in den Poren im Zahnschmelz und den Tubuli des Dentins sowie durch physikalischen Verschluss der Tubuli eine Blockierung des Stofftransports und der Weiterleitung von Reizen in den Tubuli und eine daraus resultierende Desensibilisierung der Zähne ermöglichen. Durch diese Remineralisierung des Dentins soll zum einen die Dentinhypersensibilität vermindert werden zum anderen die ursprüngliche Zahnstruktur nach einer kariogenen Belastung erhalten bleiben und / oder sich wieder regenerieren. Durch die Remineralisierung des Schmelzes durch Ablagerung der Dentalpartikel auf der Oberfläche und in den Poren (Mikrokanälchen) des Schmelzes soll ebenso die ursprüngliche Zahnstruktur nach einer kariogenen Belastung erhalten bleiben und / oder sich wieder regenerieren. Dadurch soll auch anderen Zahnerkrankungen, die auf Defekten der Mineralstruktur beruhen, wie beispielsweise Erosionen und dergleichen, vorgebeugt werden.

Die WO 00/59460 A beschreibt Kieselgelpartikel mit Partikelgrößen zwischen 0,05 und 3 µm. Die Kieselgelpartikel sind in einer dentalen Zusammensetzung in einer Menge von 0,5 bis 15 Gew.-% enthalten. Die dentale Zusammensetzung enthält zudem wasserlösliche desensibilisierende Zusätze und andere Zusätze. Mit den Kieselgelpartikeln sollen die Tubuli blockiert werden, so dass Flüssigkeitsströmungen unterbunden werden. Die Partikel des Kieselgels oder der Fällungskieselsäure sind nicht sphärisch und sie sind porös.

Diese Aufgabe wird durch die Merkmale der Ansprüche 1 und 12 gelöst. Vorteilhafte Weiterbildungen der Erfindung werden in den Unteransprüchen angegeben.

Die Erfindung sieht demgemäß vor, unporöse, sphärische bzw. kugelförmige, insbesondere monodisperse, durch einteilige Kombination in Form von Beschichtung oder Co-Kondensation oder Mischung mit einem Kombinationsagens ausgewählt aus mindestens einem wasserunlöslichen Phosphat der Metalle der II. und III. Gruppe des Periodensystems sowie Zink und Zinn oder mit mindestens einer wasserunlöslichen sauerstoffhaltigen Verbindung, einem Hydroxid, Hydrogencarbonat oder Carbonat der gleichen Gruppen erzeugte, sphärische, unporöse Kieselgelpartikel, insbesondere definierter einheitlicher Größe enthaltende und insbesondere therapeutisch wirkende Dentalpartikel einer Partikelgröße zwischen 0,1 und 2 µm, insbesondere zwischen 0,5 und 1,5 µm, herzustellen und in eine dentale Zusammensetzung einzubringen, und zwar lediglich in einer SiO₂-Menge - resultierend vom Kieselgel - unter 1 Gew.-%, insbesondere von 0,1 bis 0,9 Gew.-%, ganz besonders von 0,3 bis 0,7 Gew.-%. Wasserunlöslich im Sinne der vorliegenden Erfindung bedeutet keine absolute Wasserunlöslichkeit, sondern eine Schwerlöslichkeit, bei der unter physiologischen Bedingungen geringe Mengen an Ionen für die beschriebenen Prozesse der Remineralisierung zur Verfügung gestellt werden können.

Im Falle der Beschichtung und der Co-Kondensation ist die Herstellung derartiger Dentalpartikel bekannt. Die dritte Herstellungsvariante für die einteilige Kombination sieht eine Mischung von Teilchen zu Granalien vor, wobei vorzugsweise jeweils monodisperse Teilchen des Kieselgels mit z. B. gleich großen monodispersen Teilchen des Phosphats, einer wasserunlöslichen sauerstoffhaltigen Verbindung, einem Hydroxid, Hydrogencarbonat oder Carbonat in z. B. einem Granulier-Mischer miteinander vermengt und granuliert werden. Dabei werden Teilchengrößen zwischen 0,05 und 1,2 µm, insbesondere zwischen 0,1 und 1,0 µm, miteinander vermengt und durch Granulieren Dentalpartikel der oben angegebenen Korngröße hergestellt.

Vorzugsweise wird mindestens ein Apatit, insbesondere der Metalle Calcium, Magnesium, Strontium und Barium, insbesondere die Hydroxyl-Apatite mit den Kieselgelpartikeln zu Dentalpartikeln kombiniert. Zudem eignen sich z. B. die folgenden Phosphate: Fluor-Apatit, Chlor-Apatit, Tri-Calciumphosphat und andere auf Calciumphosphat basierende Verbindungen wie Monocalciumphosphat, Tricalciumphosphat und Tetracalciumphosphat. Weitere zweckmäßige Kombinationen ergeben sich mit den genannten sauerstoffhaltigen Verbindungen wie z.B MgO, SrO, BaO, Al₂O₃, ZnO, SnO und SnO2, aber auch Boraten, Aluminaten etc. sowie den Hydroxiden, Hydrogencarbonaten und Carbonaten dieser Metalle.

Die Dentalpartikel, insbesondere die Hydroxylapatite, Phosphate, sauerstoffhaltigen Verbindungen, Hydroxide, Hydrogencarbonate und Carbonate aufweisenden Dentalpartikel, sind in der fertigen Dentalzusammensetzung in Mengen z. B. von 1 bis 5 Gew.-%, enthalten. Die Dentalpartikel selbst tragen bzw. enthalten die SiO₂-Anteile aus dem Kieselgel in Mengen von z. B. 10 bis 90 Gew.-%, insbesondere von 30 bis 70 Gew.-%.

Fehlende Bestandteile bis jeweils 100 % in den Dentalpartikeln bestehen jeweils aus den anderen oben genannten desensibilisierenden Agentien wie Hydroxyl-Apatit, Strontium-Hydroxyl-Apatit, Magnesium-Hydroxyl-Apatit oder den anderen Phosphaten, den sauerstoffhaltigen Verbindungenn, den Hydroxiden, Hydrogencarbonaten oder Carbonaten der Metalle der II. und III. Hauptgruppe des Periodensystems sowie Zink und Zinn.

Vorzugsweise werden Dentalpartikel mit einem Schüttgewicht zwischen 1,0 und 4,0, insbesondere 1,5 und 3 g/cm³ erzeugt. Das Schüttgewicht wurde bestimmt nach der Vorschrift der DIN 53 912.

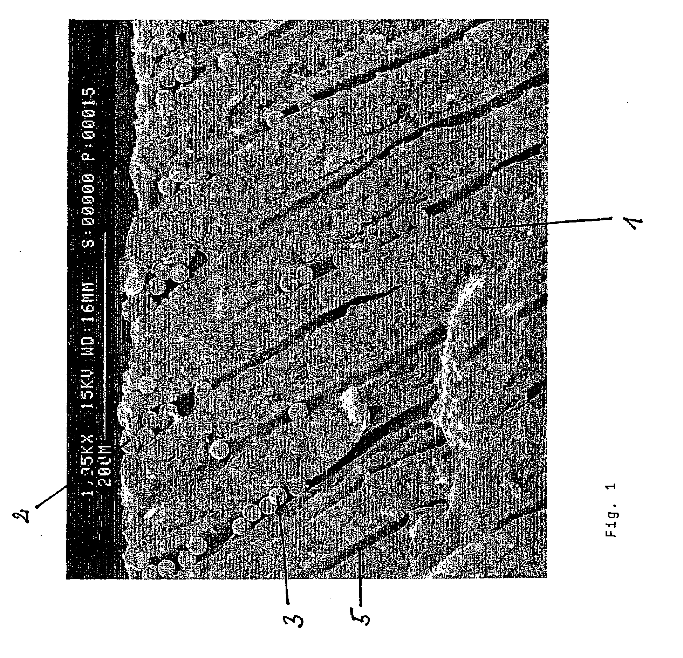

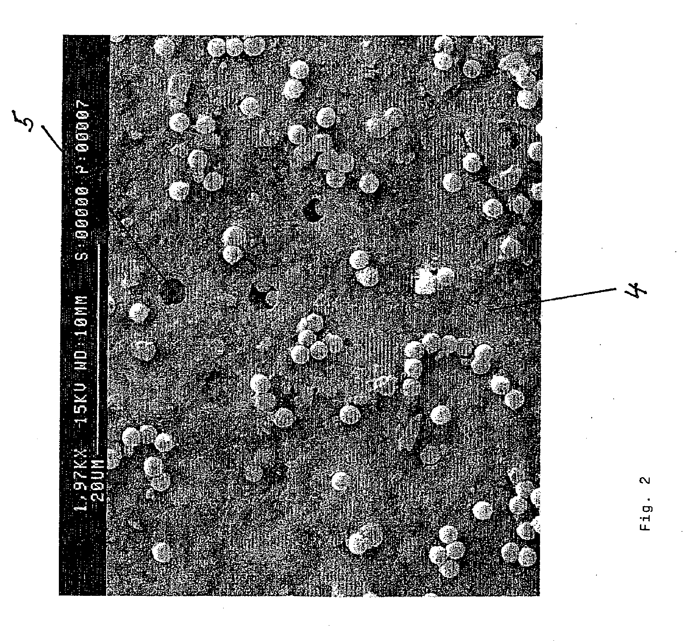

Es hat sich gezeigt, dass erfindungsgemäße dentale Zusammensetzungen durch das Zusammenwirken des Kieselgels mit dem Kombinationsmaterial eine regelmäßige An- oder Einlagerung der sphärischen Dentalpartikel an die Zahnoberfläche oder in die Zahnstruktur und in die Dentin-Tubuli ergeben, die durch die getrennte Verwendung z. B. der Phosphate oder Kieselgelpartikel oder in Kombination mit anderen Substraten nicht erreichbar ist. Die regelmäßige Ein- und Anlagerung der sphärischen Partikel - wie sie z. B. in den Fig. 1 und 2 erkennbar ist, erfolgt offenbar durch die Wechselwirkung mit der Oberflächenladung des Zahns, die durch die van-der-Waalschen Kräfte und die ionische Bindung charakterisiert ist, sowie durch die Wechselwirkung mit der Oberflächenladung der Tubuli und durch die Wechselwirkung mit der extrazellulären Dentinflüssigkeit sowie durch eine Kombination aller drei Wirkungen. In jedem Fall ergibt sich eine überraschend starke Immobilisierung bzw. Haftung der Dentalpartikel, die eine Voraussetzung für die Einwirkung auf die geschädigten Zahnregionen ist.

Fig. 1 zeigt einen Schnitt durch den Dentin 1 eines Zahns sowie an- und eingelagerte, erfindungsgemäß verwendete Dentalpartikel 2, 3 sowie Tubuli 5.

Fig. 2 zeigt eine Draufsicht auf einen Teilbereich einer Dentinoberfläche 4 eines Zahns mit offenen Tubuli 5.

Die Einzigartigkeit der Ladung der erfindungsgemäßen sphärischen Dentalpartikel wird durch die Geometrie der sphärischen Partikel, die einen piezoelektrischen Effekt hervorruft, sowie durch die ionischen mit den SiO2-Teilchen kombinierten Substanzen, die die sphärischen Dentalpartikel aufweisen, hervorgerufen.

Überraschenderweise optimieren niedrige Konzentrationen von Silica d.h. weniger als 1 Gew. % in der fertigen Zusammensetzung die elektrostatische Wechselwirkung mit der entsprechenden dentalen Oberfläche. Dies wird auf die Geometrie der sphärischen Dentalpartikel zurückgeführt, die einen elektrostatischen Effekt hervorrufen, bei dem höhere Konzentrationen von Silica die elektrostatische Wechselwirkung der Dentalpartikel mit den Zahnoberflächen verringern.

Zusätzlich zur elektrostatischen Wechselwirkung stellen die Kationen, die in den Dentalpartikeln vorliegen, ein Reservoir oder einen Kristallisationskeim für die Bildung von kationenabhängigen Formen von Hydroxylapatit dar. Die Kationenabhängigkeit zeigt sich darin, dass sich Calcium beispielsweise bevorzugt auf der Zahnoberfläche ablagert, wohingegen andere Kationen wie Strontium und Magnesium sich bevorzugt innerhalb der Dentintubuli ablagern.
Die sphärischen Dentalpartikel werden an der Oberfläche der jeweiligen Zahnsubstanz durch ihre inhärente Ladung, wie oben beschrieben, festgesetzt. Die sphärischen Dentalpartikel füllen dabei auch Lücken in den Schmelz-/Dentinoberflächen. Darüber hinaus ergibt sich eine physische Migration der sphärischen Dentalpartikel in die Schmelzporen und die Dentintubuli. Die sphärischen Dentalpartikel mit ihrer einzigartigen Geometrie und Ladungscharakteristik haften und verbleiben in den Schmelzporen und Dentintubuli. Durch ihren Verbleib in den Tubuli interagieren die sphärischen Dentalpartikel mit dem Dentinfluid durch Adsorption des Fluids an ihrer Oberfläche. Dadurch wird die Turnoverrate des Dentinfluids eingeschränkt oder vermindert. Durch die Verminderung des Flusses der Dentinflüssigkeit aufgrund von Veränderungen an der Oberfläche durch externe Quellen werden die Zellkörper oder die Fortsätze der Odontoblasten durch die externen Reize nicht gereizt und lösen daher nicht das Aktionspotential der Pulpanerven aus.

Die Auswahl der sphärischen Dentalpartikel gewährleistet aufgrund der Kugelform eine dichteste Kugelpackung zum Verschließen der Schmelzporen und der Tubuli, insbesondere bei erfindungsgemäßer Verwendung von sphärischen Dentalpartikeln unterschiedlichen Durchmessers, sowie die oben erwähnte günstige elektrische Ladungsverteilung auf der Oberfläche der Dentalpartikel, die insbesondere die Immobilisierung gewährleistet.

Es liegt im Rahmen der Erfindung die Dentalpartikel mit unterschiedlichen Durchmessern für die dentalen Zusammensetzungen zu kombinieren. Es liegt auch im Rahmen der Erfindung, Dentalpartikel, die unterschiedliche Kombinationsagentien aufweisen, in einer dentalen Zusammensetzung zu verwenden, wobei dabei auch Dentalpartikel unterschiedlichen Durchmessers eingesetzt werden können.
Die Partikel sind einzigartig bezüglich ihrer Dichte und Geometrie, die eine Penetration in Poren und Tubuli sicherstellt und die eine gewisse Verweilzeit mit sich bringen, so dass die Partikel als Keim für die Wirksamkeit in der Form einer Desensibilisierung von schmerzempfindlichen Zähnen als auch einer Remineralisierung der Zahnoberfläche zum Erhalt der Oberflächenintegrität dienen.

Die unterschiedlichen Durchmesser und die unterschiedliche Dichte der sphärischen Partikel sind in Einklang mit den unterschiedlichen Porengrößen der Dentin- / Schmelzoberfläche in Abhängigkeit von der Tiefe des Eindringens der Noxe, dem Alter des Individuums und der Stellung des Zahns im Gebiss.

Die Erfindung unterscheidet sich vom Stand der Technik dadurch, dass herausgefunden wurde, dass sowohl die Geometrie der Partikel als sphärische Partikel als auch die Bandbreite der Partikelgröße dergestalt zu optimieren ist, dass die unterschiedlichen Größen der Öffnungen in der Zahnoberfläche berücksichtigt werden. Die erfindungsgemäßen Partikel können die Oberfläche durchdringen und erreichen dadurch eine ausreichend lange Verweilzeit in den Tubuli und Poren, die notwendig ist für den Prozess der Remineralisierung, und erreichen daher ihre therapeutische Wirksamkeit.

## Patentansprüche

1. Sphärische unporöse Dentalpartikel, insbesondere für remineralisierende und gegen schmerzempfindliche Zähne wirksame dentale Zusammensetzungen wie Zahnpasta, Zahnpulver, Mundwasser, Kaugummi, zahnärztliche Formulierungen oder dergleichen, aufweisend
a) Kieselgel mit
b) Partikelgrößen zwischen 0,1 und 2 µm
c) in einteiliger Kombination mit
d) mindestens einem wasserunlöslichen Kombinationsagens
e) das unter physiologischen Bedingungen geringe Mengen an Ionen für eine Remineralisierung des Zahnmaterials zur Verfügung stellt
f) und ausgewählt ist aus mindestens
einem Phosphat,
einem Oxid,
einer anderen sauerstoffhaltigen Verbindung in Form von Boraten, Aluminaten,
einem Hydroxid,
einem Hydrogencarbonat oder
einem Carbonat
der Metalle der II. und III. Gruppe des Periodensystems, sowie Zink und Zinn,
g) die Dentalpartikel die SiO₂-Anteile aus dem Kieselgel in Mengen von 10 bis 90 Gew.-% enthalten, wobei fehlende Bestandteile in den Dentalpartikeln bis jeweils 100% aus den Kombinationsagentien bestehen.

2. Dentalpartikel nach Anspruch 1,
**dadurch gekennzeichnet, dass** die Partikelgrößen zwischen 0,5 und 1,5 µm liegen.

3. Dentalpartikel nach Anspruch 1 und/oder 2,
**gekennzeichnet durch**
eine einteilige Beschichtungs- oder Co-Kondensation- oder durch Mischung und Granulierung-Kombination von Kieselgel mit dem mindestens einem Kombinationsagens.

4. Dentalpartikel nach einem oder mehreren der Ansprüche 1 bis 3,
**dadurch gekennzeichnet, dass** die Dentalpartikel in monodisperser Form vorliegen.

5. Dentalpartikel nach einem oder mehreren der Ansprüche 1 bis 4,
**dadurch gekennzeichnet, dass** in den beschichteten oder zusammengemischten Dentalpartikeln die Partikelgröße der Kieselgelpartikel zwischen 0,05 und 1,5, insbesondere zwischen 0,1 und 1,0 µm beträgt.

6. Dentalpartikel nach einem oder mehreren der Ansprüche 1 bis 5,
**dadurch gekennzeichnet, dass** in den zusammengemischten granulierten Dentalpartikeln die Kieselgelpartikel und die Phosphatpartikel gleich groß sind oder in unterschiedlichen Partikelgrößen vorliegen.

7. Dentalpartikel nach Anspruch 5 und/oder 6,
**dadurch gekennzeichnet, dass** die Kieselgelpartikel in monodisperser Form vorliegen.

8. Dentalpartikel nach einem oder mehreren der Ansprüche 5 bis 7,
**dadurch gekennzeichnet, dass** das Kombinationsagens in der granulierten Kombination mindestens ein Apatit, insbesondere Hydroxylapatit, ein Oxid, eine andere sauerstoffhaltige Verbindung, ein Hydroxid, Hydrogencarbonat oder ein Carbonat ist.

9. Dentalpartikel nach einem oder mehreren der Ansprüche 1 bis 8,
**dadurch gekennzeichnet, dass** der SiO₂-Anteil in den Dentalpartikeln 10 bis 90, insbesondere 30 bis 70 Gew.-% beträgt.

10. Dentalpartikel nach einem oder mehreren der Ansprüche 1 bis 9,
**dadurch gekennzeichnet, dass** das Schüttgewicht der Dentalpartikel 1,0 bis 4,0 g/cm³, insbesondere 1,5 bis 3,0 g/cm³ beträgt.

11. Dentalpartikel nach einem oder mehreren der Ansprüche 1 bis 10,
**dadurch gekennzeichnet, dass** das Phosphat mindestens ein Apatit, insbesondere der Metalle Ca, Mg, Sr, Ba, Aluminium, Bor sowie Zink und Zinn, insbesondere Hydroxylapatit ist.

12. Dentale, insbesondere remineralisierende und gegen schmerzempfindliche Zähne wirksame Zusammensetzung wie Zahnpasta, Zahnpulver, Mundwasser, Kaugummi, zahnärztliche Formulierungen oder dergleichen, aufweisend Dentalpartikel nach einem oder mehreren der Ansprüche 1 bis 11, wobei die Dentalpartikel in einer Menge enthalten sind, dass die SiO₂-Menge, resultierend aus dem Kieselgel, unter 1 Gew.-% beträgt und in Mengen von 0,1 bis 10 Gew.-% enthalten sind.

13. Zusammensetzung nach Anspruch 12,
**dadurch gekennzeichnet, dass** die SiO₂-Menge von 0,1 bis 0,9, insbesondere von 0,3 bis 0,7 Gew.-% beträgt.

14. Zusammensetzung nach einem oder mehreren der Ansprüche 12 und/oder 13,
**dadurch gekennzeichnet, dass** die Dentalpartikel in der dentalen zusammensetzung in Mengen zwischen 1 und 5 Gew.-% enthalten sind.

15. Verwendung von Dentalpartikeln nach einem oder mehreren der Ansprüche 1 bis 11 mit unterschiedlichen Durchmessern und/oder mit unterschiedlichen Kombinationsagentien in einer dentalen Zusammensetzung nach einem oder mehreren der Ansprüche 12 bis 14.

## Claims

1. Spherical, non-porous dental particles notably for dentin remineralizing and desensitizing compositions like toothpaste, tooth powder, mouthwash, chewing gum, professional formulations etc comprising
a) silica with
b) a particle size between 0.1 and 2 µm
c) in combination with
d) at least one water insoluble combination agent
e) which releases under physiological conditions small amounts of ions for a remineralisation of the dental material
f) chosen from at least
a phosphate,
an oxide
another oxygen containing compound
a hydroxide
a hydrogen carbonate or
a carbonate of the metals of the II. and III. group of the periodic system as well as zinc and tin
g) the dental particles containing the Si02 from the silica in amounts of 10 % to 90% where missing percentages to 100 % stem from the combination agents.

2. Dental particle of claim 1 **characterized by** having a particle size between 0.5 and 1.5 µm.

3. Dental particle of claims 1 and / or 2 **characterized by** coating, cocondensation or mixing and granulating of the silica particles with at least one combination agent.

4. The dental particle of one or more of the claims 1 to 3 **characterized by** being mono-disperse.

5. Dental particle of one or more of the claims 1 to 4 **characterized by** having a particle size of the coated or mixed silica particles between 0.05 and 1.5 µm notably between 0.1 and 1.0 µm.

6. Dental particle of one or more of the claims 1 to 5 **characterized by** the silica and phosphate particles being of the same or of different sizes within in the mixed and granulated particles.

7. Dental particle of one or more of the claims 5 and /'or 6 **characterized by** the silica particles being mono-disperse.

8. Dental particle of one or more of the claims 5 to 7 **characterized by** the combination agent being at least one apatite and notably a hydroxyapatite, an oxide, another oxygen containing agent, a hydroxide or a carbonate.

9. Dental particle of one or more of the claims 1 to 8 **characterized by** the amount of silica in the dental particles being between 10 and 90 % notably between 30 and 70% by weight.

10. Dental particle of one or more of the claims 1 to 9 **characterized by** the bulk density of the dental particles being between 1.0 and 4.0 g/cm³ notably between 1.5 and 3.0 g/cm³.

11. Dental particles of one or more of the claims 1 to 10 **characterized by** the phosphate being at least one apatite notably of the metals Ca, Mg, Sr, Ba, Al, B, Zn and Sn, notably a hydroxyapatite.

12. Dental notably dentin remineralizing and desensitizing compositions like toothpaste, tooth powder, mouthwash, chewing gum, professional formulations etc comprising dental particles of one or more of the claims 1 to 11 where the dental particles are contained in an amount that the amount of silica from the silica particles is below 1 % by weight and are contained in an amount of 0.1 to 10% by weight.

13. Composition of claim 12 **characterized by** the amount of silica being between 0.1 and 0.9 % notably 0.3 to 0.7 % by weight.

14. Composition of one or more of the claims 12 and / or 13 **characterized by** the dental particles being contained in an amount of 1 to 5 % by weight.

15. The use of dental particles of one or more of the claims 1 to 11 with different diameters and / or different combination agents in a dental composition of one or more of the claims 12 to 14.

## Revendications

1. Particules dentaires sphériques non poreuses, en particulier pour les compositions dentaires reminéralisantes et efficaces en cas de dents sensibles, telles que les dentifrices, les poudres dentaires, les solutés pour bains de bouche, les chewing-gums, les formulations de médecine dentaire ou des produits analogues, contenant
a) du gel de silice
b) avec une taille des particules comprise entre 0,1 et 2 µm
c) en combinaison d'une seule partie avec
d) au moins un agent de combinaison insoluble dans l'eau
e) qui met à disposition dans les conditions physiologiques des quantités minimes d'ions pour la reminéralisation du matériel dentaire
f) et est sélectionné à partir d'au moins
un phosphate
un oxyde
un autre composé contenant de l'oxygène sous forme de borates, d'aluminates
un hydroxyde
un bicarbonate ou
un carbonate
des métaux des II et III groupe du système périodique ainsi que du zinc et de l'étain
g) les particules dentaires qui contiennent des proportions de SiO₂ du gel de silice allant de 10 à 90 poids/%, les composants éventuellement absents des particules dentaires étant remplacés à chaque fois jusqu'à 100 % par les agents de combinaison.

2. Des particules dentaires conformes à la revendication 1,
**caractérisées par le fait que**
la taille des particules est comprise entre 0,5 et 1,5 µm.

3. Des particules dentaires conformes à la revendication 1 et/ou 2,
**caractérisées par** une combinaison d'un revêtement en une partie ou d'une cocondensation ou d'une combinaison d'un mélange et d'une granulation de gel de silice avec au moins un agent de combinaison.

4. Des particules dentaires conformes à la revendication 1 à 3,
**caractérisées par le fait que** les particules sont disponibles sous une forme monodispersée.

5. Des particules dentaires conformes à une ou à plusieurs des revendications 1 à 4,
**caractérisées par le fait que** la taille des particules de gel de silice est comprise entre 0,05 et 1,5, en particulier entre 0,1 et 1 µm.

6. Des particules dentaires conformes à une ou à plusieurs des revendications 1 à 5,
**caractérisées par le fait que** les particules de gel de silice et les particules de phosphate présentent la même taille pour les particules dentaires mélangées et granulées ou sont présentes selon des tailles différentes.

7. Des particules dentaires conformes aux revendications 5 et/ou 6,
**caractérisées par le fait que** les particules sont disponibles sous une forme monodispersée.

8. Des particules dentaires conformes à une ou à plusieurs des revendications 5 à 7,
**caractérisées par le fait que** l'agent de combinaison, contenu dans la combinaison granulée est au moins une apatite, en particulier une hydroxyapatite, un oxyde, un autre composé contenant de l'oxygène, un hydroxyde; un bicarbonate ou un carbonate.

9. Particules dentaires conformes à une ou plusieurs des revendications 1 à 8,
**caractérisées par le fait que** le pourcentage de SiO₂ dans les particules dentaires s'élève de 10 à 90, en particulier de 30 à 70 poids/%.

10. Particules dentaires conformes à une ou plusieurs des revendications 1 à 9,
**caractérisées par le fait que** la densité apparente des particules dentaires est de 1,0 à 4,0 g/cm³, en particulier 1 à 3,0 g/cm³.

11. Particules dentaires conformes à une ou plusieurs des revendications 1 à 10,
**caractérisées par le fait que** le phosphate est au moins une apatite, en particulier des métaux Ca, Mg, Sr, Ba, aluminium, bore, zinc et étain, en particulier une hydroxyapatite.

12. Composition dentaire, en particulier pour les compositions dentaires reminéralisantes et efficaces telles que les dentifrices, les poudres dentaires, les solutés pour bains de bouche, les chewing-gums, les formulations de médecine dentaire ou des produits analogues, en cas de dents sensibles, présentant des particules dentaires selon une ou plusieurs des revendications 1 à 11, la particule dentaire étant composée sur le plan quantitatif de telle façon que la quantité de SiO₂, résultant du gel de silice, est inférieure à 1 poids/% et est présente en quantités allant de 0,1 à 10 poids/%.

13. Composition selon la revendication 12,
**caractérisée par le fait que** la quantité de SiO₂ est de 0,1 à 0,9, en particulier de 0,3 à 0,7 poids/%.

14. Composition selon une ou plusieurs des revendications 12 et/ou 13,
**caractérisée par le fait que** les particules dentaires constituent pour la composition dentaire des quantités allant de 1 à 5 poids/%.

15. Utilisation de particules dentaires conformes aux revendications 1 à 11, comportant des diamètres variables et/ou des agents de combinaisons différents pour une composition dentaire selon les droits 12 à 14.
